# EUROPEAN PATENT SPECIFICATION

(11) **EP 3 071 338 B1**
(45) Date of publication and mention of the grant of the patent: **11.05.2022**
(21) Application number: 14799401.6
(22) Date of filing: 14.11.2014
(51) Int. Cl.: B06B 1/02

(54) **ULTRASOUND TRANSDUCER ASSEMBLY**
ULTRASCHALLWANDLERANORDNUNG
ENSEMBLE TRANSDUCTEUR À ULTRASONS

(30) Priority: 18.11.2013 US 201361905409 P; 03.01.2014 EP 14150094
(43) Date of publication of application: 28.09.2016
(73) Proprietor: Koninklijke Philips N.V., 5656 AG Eindhoven (NL)
(72) Inventor: BROCK-FISHER, George Anthony, NL-5656 AE Eindhoven (NL); SAVORD, Bernard Joseph, NL-5656 AE Eindhoven (NL)
(74) Representative: Philips Intellectual Property & Standards
(86) International application number: PCT/EP2014/074553
(87) International publication number: WO 2015/071387

(56) References cited:
- WO-A1-2013/111063
- US-A1- 2009 082 673
- US-A1- 2012 256 519
- US-A1- 2012 299 439
- US-A1- 2013 064 043
- US-A1- 2013 169 110
- US-B2- 7 892 175

## Description

### FIELD OF THE INVENTION

The present invention relates to an ultrasound transducer assembly, in particular for ultrasound imaging systems.

### BACKGROUND OF THE INVENTION

In the field of ultrasound transducers and in particular in the field of ultrasound imaging systems, it is generally known to use micro-machined ultrasound transducer elements (CMUTs). Due to the fact that the capacitive micro-machined transducer elements can be fabricated by semiconductor processing techniques, the size of the transducers can be shrinked to very small feature sizes.

Further, it is generally known to drive the ultrasound transducers including the micro-machined ultrasound transducer arrays by means of application specific integrated circuits, which serve as an electrical interface to each of the transducer elements.

For integrating the whole transducer assembly, it is further known to fabricate the transducer array directly on a top surface of the application specific integrated circuits or to attach the transducer arrays and the application specific integrated circuits to each other in order to reduce the overall size of the transducer assembly. In order to reduce the technical effort for connecting the electrodes of each of the transducer elements to the application specific integrated circuit, usually the top electrodes, which are attached to the flexible membrane of the transducer elements are each electrically connected to each other and connected to a voltage supply for applying a bias voltage to these electrodes. The second electrodes of the transducer elements, which are disposed at a bottom of a cavity of the transducer elements are each individually connected to the application specific integrated circuit below the transducer array in order to apply individual driving signals to each of the transducer elements and to receive ultrasound signals to be detected and to be evaluated. The disadvantage of the transducer array in combination with the application specific integrated circuit as driver device is that a large parasitic capacitance is formed between the bottom electrode of the transducer elements and the top metal layer of the application specific integrated circuit This parasitic capacitance can be a significant fraction of the cMUT capacitance itself and must be charged during excitation of the cMUT device resulting in a low overall transmit efficiency. Furthermore, on signal reception, the parasitic capacitance shunts desired receive signals from entering the circuitry. This results in poor signal to noise ratio when receiving signals.

To reduce the parasitic capacitance between the transducer array and the driver circuit, US 2013/0088118 A1 proposes to include additional floating electrodes between the first and the second electrode of each of the transducer elements within the cavity. The disadvantage of this solution to reduce the parasitic capacitance is that the transducer array has a complicated structure and the technical effort for manufacturing those transducer arrays is increased.

US 2013/169110 discloses an ultrasonic transducer structure which includes a driving wafer that includes a driving circuit; and an ultrasonic transducer wafer that is disposed on the driving wafer and includes a first wafer in which a via-hole is formed. A first insulating layer is formed on the first wafer, a second wafer spaced apart from the first insulating layer, and a cavity is formed between the first insulating layer and the second wafer.

### SUMMARY OF THE INVENTION

It is an object of the present invention to provide an improved ultrasound transducer assembly, in particular for ultrasound imaging systems having an increased transmit efficiency and an increased receive signal to noise ratio with low technical effort.

According to one aspect of the present invention, an ultrasound transducer assembly, in particular for ultrasound imaging systems, is provided comprising:
- a transducer array including a plurality of transducer elements for emitting and receiving ultrasound waves, wherein each of the transducer elements includes a first electrode connected to a flexible membrane and a second electrode,
- an integrated circuit device connected to the transducer array for driving the transducer elements,
- wherein the first electrodes are coupled to the integrated circuit device for providing an alternating drive voltage to each of the transducer elements and wherein the second electrodes are electrically connected to each other and coupled to a voltage supply for providing a bias voltage to the transducer elements, and
wherein the first electrodes are each connected by means of a via to the integrated circuit device and the vias are fed through the second electrodes.

In a further aspect of the present invention, an ultrasound imaging system is provided including an ultrasound transducer assembly of this kind for emitting and receiving ultrasound waves.

Preferred embodiments of the invention are defined in the dependent claims. It shall be understood that the claimed method has similar and/or identical preferred embodiments as the claimed device and as defined in the dependent claims.

The present invention is based on the idea to reduce the parasitic capacitance by inverting the electrical connections to the transducer elements. This is provided by connecting the first electrode of each of the transducer elements which are connected to the flexible membrane to the integrated circuit device by means of vias through the second electrodes for driving each of the first electrodes individually and by connecting the second electrodes of the transducer elements to each other and to a common voltage supply in order to provide a necessary bias voltage to these second electrodes. Hence, the first electrode is used as the active electrode and is connected to the driver and/or receiver device of the integrated circuit device so that the second electrode is the passive electrode. This configuration reduces the parasitic capacitance of the connection to the active electrode since the active electrodes are top electrodes and the driver and/or receiver device is connected to the active electrodes by means of vias fed through the second electrodes. Hence, the overall transmit efficiency and the signal to noise ratio of the transducer array can be significantly improved. Additionally, the overall size of the transducer assembly is reduced.

In a preferred embodiment, the first electrodes are top electrodes of the transducer array. This is a possibility to transmit and receive ultrasound waves with low attenuation, since the ultrasound waves are directly emitted and received so that the overall signal strength is increased.

It is further preferred that the first electrodes are each coupled to a top surface of the flexible membranes. This is a possibility to reduce the technical effort for manufacturing the transducer elements, since the electrodes can be formed on the flexible membranes, e.g. by means of a deposition process.

In a preferred embodiment, the second electrodes are bottom electrodes attached to an isolation layer. This is a simple possibility to provide a bias electrode isolated from the integrated circuit device.

In a preferred embodiment, the vias are fed through the isolation layer. This is a possibility to directly contact the first electrodes to driver elements of the integrated circuit disposed below the respective transducer element.

In a preferred embodiment, the second electrodes are rigid electrodes. This is a possibility to provide the second electrodes as bias electrodes with low technical effort, since the rigid electrodes can be manufactured with low technical effort, e.g. by means of a deposition method.

In a preferred embodiment, the cavity is formed between each of the first electrodes and each of the second electrodes, respectively. This is a possibility to easily transmit and receive ultrasound waves since the flexible membrane can be deflected into the cavity.

In a preferred embodiment, the flexible membranes of the transducer elements are each supported by means of a spacer. This is a simple possibility to form the flexible membranes in a distance to the second electrode so that the flexible membranes and the first electrodes can easily be deflected into the cavity.

It is preferred that the vias for connecting the first electrode to the integrated circuit device are integrated in the spacer. This is a further possibility to reduce the overall size of the transducer array.

In a preferred embodiment, the transducer elements are capacitive micro-machined ultrasound transducer elements. This is a possibility to form the transducer array with low technical effort and with a small overall size since the transducer array can be manufactured in an IC process in/on a silicon wafer.

In a preferred embodiment, the integrated circuit device is an application specific integrated circuit. This is a possibility to connect the transducer array directly to a specific driving element, since the application specific integrated circuit is designed to drive the transducer elements and to receive signals according to the received ultrasound waves.

In a preferred embodiment, the transducer elements are monolithically formed on the integrated circuit device. This is a possibility to manufacture the ultrasound transducer assembly with low technical effort and with reduced feature sizes.

In a further preferred embodiment, the integrated circuit device and the transducer elements are monolithically integrated on a substrate. This is a further possibility to reduce the manufacturing time and effort since almost the whole device can be formed monolithically e.g. by means of micro-electronic process technology. An additional advantage of this embodiment is that the transducer elements can be manufactured on a premanufactured integrated circuit device. As mentioned above, by connecting the top electrode to the integrated circuit for providing AC driving signals to the respective transducer element and by connecting the bottom electrode to a common DC power supply for providing a DC bias voltage to the transducer array, the parasitic capacitance between the driven or excited electrode of the transducer elements can be significantly reduced so that the transmit efficiency and receive signal to noise ratio of the transducer array can be significantly improved and the quality of the analytic information received from the ultrasound transducer assembly e.g. the image data can be improved.

### BRIEF DESCRIPTION OF THE DRAWINGS

These and other aspects of the invention will be apparent from and elucidated with reference to the embodiment(s) described hereinafter. In the following drawings
Fig. 1 shows a schematic illustration of an ultrasound imaging system;
Fig. 2 shows a schematic sectional view of an ultrasound transducer array and an integrated circuit device according to the state of the art; and
Fig. 3 shows a schematic sectional view of an ultrasound transducer array and an integrated circuit according to the present invention.

### DETAILED DESCRIPTION OF THE INVENTION

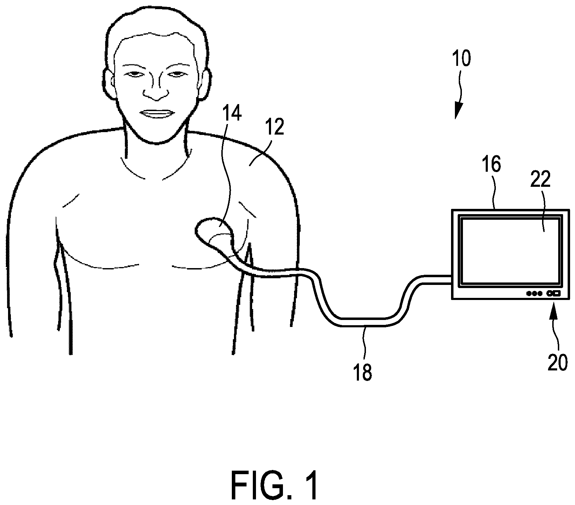

Fig. 1 illustrates a principle design of an ultrasound imaging system generally denoted by 10. This figure is used to explain the background of the ultrasound imaging. It shall be understood that the claimed ultrasound transducer assembly is not restricted to such kind of applications.

The ultrasound imaging system 10 is used for scanning an area or a volume of a body, e.g. of a patient 12. It shall be understood that the ultrasound system 10 may also be used for scanning other areas or volumes, e.g. body parts of animals or other living beings.

For scanning the patient, an ultrasound probe 14 is provided. In the embodiment shown in Fig. 1, the ultrasound probe 14 is connected to a console device 16. The console device 16 is shown as a mobile console. This console 16 may however also be realized as a stationary device. The console device 16 is connected to the probe 14 via an interface 18 formed as a via. Further, the console device 16 comprises an input device 20 to enable the user to control the ultrasound imaging system 10 and may comprise a display 22 to display data and images generated by the ultrasound imaging system 10. By this, the volume within the patient 12 that is scanned via the ultrasound probe 14 can be viewed on the console device 16 by the user of the ultrasound imaging system 10.

The ultrasound probe 14 usually comprises an ultrasound transducer array driven by a driver device as described in the following in order to transmit and receive ultrasound waves and to provide ultrasound image data from the received ultrasound waves.

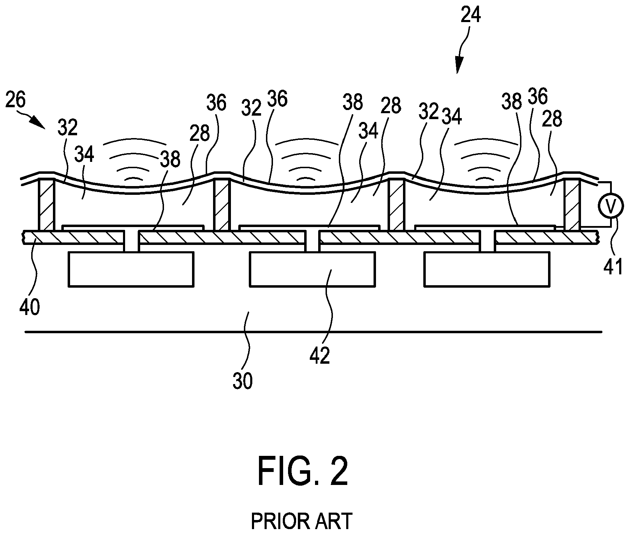

Fig. 2 shows an ultrasound transducer assembly as known from the state of the art, which is generally denoted by 24. The ultrasound transducer assembly 24 comprises a transducer array 26 of a plurality of transducer elements 28, which are formed as capacitive micro-machined ultrasound transducer (CMUT) elements. The transducer array 26 is connected to a top surface of an application specific integrated circuit 30. The transducer elements 28 comprises a flexible membrane 32, a cavity 34 and a top electrode 36 and a bottom electrode 38. The top electrode 36 is formed on or embedded in the flexible membrane 32 and the bottom electrode 38 is formed at a bottom of the cavity 28 on top of or embedded in an isolation layer 40 isolating the transducer array 26 from the application specific integrated circuit 30.

The top electrodes 36 are connected to each other and are connected to a DC power supply 41 for providing a DC bias voltage to the transducer elements 28. The bottom electrodes 38 are each individually connected to a driving element 42 of the application specific integrated circuit 30 in order to provide an individual AC signal to each of the transducer elements 28 and to drive the transducer elements 28 individually. Hence, by applying the AC signal to the bottom electrode 38 and by receiving a signal from the bottom electrode 38, the application specific integrated circuit can transmit and receive ultrasound waves by each of the transducer elements 28.

The bottom electrodes 38, which are connected by vias to the application specific integrated circuit 30, and the isolation layer 40 - have parasitic capacitances parallel to the bottom electrodes 38 and the input of the application specific integrated circuit 30. The parasitic capacitances of the transducer elements 28 can be in the order of 0.5 pF, so that the transmit efficiency and receive signal to noise ratio degradation may be in the range of 2 db. These parasitic capacitances reduce the transmit efficiency and receive signal to noise ratio of the signals received from the transducer elements 28 so that the signal quality of the transducer assembly 24 is reduced.

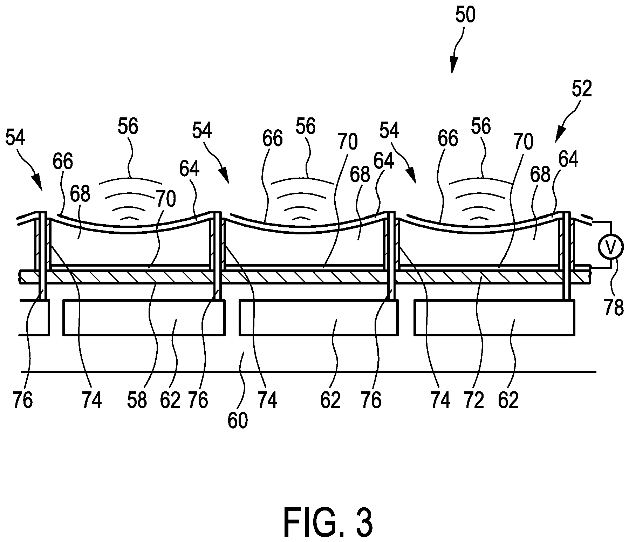

In Fig. 3, an ultrasound transducer assembly according to the present invention is shown in a schematic sectional view and generally denoted by 50. The ultrasound transducer assembly 50 comprises an ultrasound transducer array 52 having a plurality of transducer elements 54, which are preferably formed as capacitive micro-machined ultrasound transducer elements (CMUT) for emitting and receiving ultrasound waves 56. The ultrasound transducer array 52 is formed on a top surface 58 of an integrated circuit 60, which is preferably an application specific integrated circuit 60. The ultrasound transducer array 52 is preferably formed monolithically on the top surface 58 by a micro manufacturing process. The integrated circuit 60 comprises a plurality of driving and receiving elements 62 for driving the transducer elements 54 and for receiving signals from the transducer elements 54 as described in the following. The so formed ultrasound transducer assembly 50 may be used in the ultrasound probe 14 for transmitting and receiving ultrasound waves 56.

The transducer elements 54, which are formed as capacitive micro-machined ultrasound transducer element and the integrated circuit 60, which is preferably formed as an application specific integrated circuit 60 are formed or manufactured monolithically on a substrate which is preferably a semiconductor substrate by means of a micro manufacturing method e.g. a microelectronic manufacturing technology. This is a possibility to reduce the size of the ultrasound transducer assembly 50 and to reduce the technical effort for manufacturing the ultrasound transducer assembly.

The transducer elements 54 each comprise a flexible membrane 64 each including a first electrode 66, which is preferably attached to a top surface of the flexible membrane 64 or embedded in to the membrane 64. A cavity 68 is formed below the flexible membrane 64 so that the flexible membrane 64 can be deflected or excited in order to emit the ultrasound waves 56. At a bottom of the cavity 68, each transducer element 54 comprises a second electrode 70, which is coupled or embedded in to an isolation layer 72 which isolates the ultrasound transducer array 52 from the integrated circuit 60. The first electrode 66 is formed either as a flexible electrode 66 or as the rigid electrode which is coupled to the flexible membrane 64 and the second electrode 70 is formed as a rigid electrode 70. The flexible membrane 64 is separated from the second electrode 70 and the isolation layer 72 by means of support elements 74 or spacers 74.

The first electrodes are isolated from each other and each individually electrically connected to the drive elements 62 of the integrated circuit 60 by means of a via 76. The via 76 is formed within the support elements 74. Alternatively, the vias 76 may be connected laterally to the support elements 74.

The vias 76 are further fed through the isolation layer 72 and the second electrodes 70 in order to connect the first electrodes 66 individually to the integrated circuit 60. The second electrodes 70 are connected to each other and connected to a DC power supply 78 for providing a DC bias voltage to the transducer elements 54. The second electrodes 70 are preferably formed as a single metal layer deposited on a top surface of the isolation layer 72. Hence, the first electrodes 66 as the top electrodes 66 form the active electrodes and the second electrodes, which are formed as bottom electrodes, are the passive electrodes. By means of this inverse connection with the active electrode as the top electrode 66, the parasitic capacitances between the active electrode 66 and the integrated circuit 60 in general can be reduced so that the transmit efficiency and receive signal to noise ratio of the transducer elements 54 can be improved and the signal and image quality can be increased.

The cavity 68 may be a vacuum gap. The flexible membrane 64 may be deflected down to the second electrode 70 by means of the DC bias voltage in order to use the transducer elements 54 in a collapsed mode to increase the sensitivity of the ultrasound transducer assembly 50 in general. In a certain embodiment, the second electrode 70 may be covered by an additional isolation layer.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings and disclosure, within the scope of the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. An ultrasound transducer assembly (50), in particular for ultrasound imaging systems (10), comprising:
- a transducer array (52) including a plurality of transducer elements (54) for emitting and receiving ultrasound waves (56), wherein each of the transducer elements (54) includes a first electrode (66) connected to a flexible membrane (64) and a second electrode (70),
- an integrated circuit device (60) connected to the transducer array (52) for driving the transducer elements (54),
- wherein the first electrodes (66) are each connected by means of a via (76) to the integrated circuit device (60) and the vias (76) are fed through the second electrodes (70),
**characterised in that**
the second electrodes (70) are electrically connected to each other and coupled to a voltage supply (78) for providing a bias voltage between the first and second electrodes of the transducer elements (54), and
the first electrodes (66) are coupled to the integrated circuit device (60) for providing an alternating drive voltage to each of the transducer elements (54).

2. The ultrasound transducer assembly as claimed in claim 1, wherein the first electrodes (66) are top electrodes of the transducer array (52).

3. The ultrasound transducer assembly as claimed in claim 2, wherein the first electrodes (66) are each coupled to a top surface of the respective flexible membrane (64).

4. The ultrasound transducer assembly as claimed in claim 1, wherein the second electrodes (70) are bottom electrodes attached to an isolation layer (72).

5. The ultrasound transducer assembly as claimed in claim 4, wherein the isolation layer (72) is attached to a surface of the integrated circuit device (60).

6. The ultrasound transducer assembly as claimed in claim 1, wherein the second electrodes (70) are rigid electrodes.

7. The ultrasound transducer assembly as claimed in claim 1, wherein a cavity (68) is formed between each of the first electrodes and each of the second electrodes, respectively.

8. The ultrasound transducer assembly as claimed in claim 1, wherein the flexible membranes (64) of the transducer elements (54) are each supported by means of a spacer (74).

9. The ultrasound transducer assembly as claimed in claim 8, wherein the vias (76) for connecting the first electrodes (66) to the integrated circuit (60) are fed through the spacers (74).

10. The ultrasound transducer assembly as claimed in claim 4, wherein the vias (76) are fed through the isolation layer (72).

11. The ultrasound transducer assembly as claimed in claim 1, wherein the transducer elements (54) are capacitive micro-machined ultrasound transducer elements.

12. The ultrasound transducer assembly as claimed in claim 1, wherein the integrated circuit device (60) is an application specific integrated circuit.

13. The ultrasound transducer assembly as claimed in claim 1, wherein the transducer elements (54) are monolithically formed on the integrated circuit device (60).

14. The ultrasound transducer assembly as claimed in claim 1, wherein the integrated circuit device (60) and the transducer elements (54) are monolithically integrated on a substrate.

15. An ultrasound imaging system (10) including an ultrasound transducer assembly (50) as claimed in claim 1 for emitting and receiving ultrasound waves (56).

## Patentansprüche

1. Ultraschallwandler-Baugruppe (50), insbesondere für Ultraschall-Bildgebung-Systeme (10), Folgendes umfassend:
- eine Wandleranordnung (52) mit einer Vielzahl von Wandlerelementen (54) zum Aussenden und Empfangen von Ultraschallwellen (56), wobei jedes der Wandlerelemente (54) eine erste Elektrode (66), die mit einer flexiblen Membran (64) verbunden ist, und eine zweite Elektrode (70) aufweist,
- ein integriertes Schaltelement (60), das mit der Wandleranordnung (52) verbunden ist, um die Wandlerelemente (54) zu steuern,
- wobei die ersten Elektroden (66) jeweils über ein Via (76) mit dem integrierten Schaltelement (60) verbunden sind und die Vias (76) durch die zweiten Elektroden (70) geleitet werden, **dadurch gekennzeichnet, dass**
die zweiten Elektroden (70) elektrisch miteinander verbunden und mit einer Spannungsversorgung (78) gekoppelt sind, um eine Vorspannung zwischen den ersten und zweiten Elektroden der Wandlerelemente (54) bereitzustellen, und die ersten Elektroden (66) mit dem integrierten Schaltelement (60) gekoppelt sind, um eine Antriebswechselspannung für jedes der Wandlerelemente (54) bereitzustellen.

2. Ultraschallwandler-Baugruppe nach Anspruch 1, wobei die ersten Elektroden (66) obere Elektroden der Wandleranordnung (52) sind.

3. Ultraschallwandler-Baugruppe nach Anspruch 2, wobei die ersten Elektroden (66) jeweils mit einer Oberseite der jeweiligen flexiblen Membran (64) verbunden sind.

4. Ultraschallwandler-Baugruppe nach Anspruch 1, wobei die zweiten Elektroden (70) untere Elektroden sind, die an einer Isolierschicht (72) befestigt sind.

5. Ultraschallwandler-Baugruppe nach Anspruch 4, wobei die Isolierschicht (72) an einer Oberfläche des integrierten Schaltelements (60) angebracht ist.

6. Die Ultraschallwandler-Baugruppe nach Anspruch 1, wobei die zweiten Elektroden (70) starre Elektroden sind.

7. Ultraschallwandler-Baugruppe nach Anspruch 1, wobei zwischen jeder der ersten Elektroden bzw. jeder der zweiten Elektroden ein Hohlraum (68) ausgebildet ist.

8. Ultraschallwandler-Baugruppe nach Anspruch 1, wobei die flexiblen Membranen (64) der Wandlerelemente (54) jeweils mittels eines Abstandshalters (74) gehalten werden.

9. Ultraschallwandler-Baugruppe nach Anspruch 8, wobei die Vias (76) zum Verbinden der ersten Elektroden (66) mit dem integrierten Schaltelement (60) durch die Abstandshalter (74) geleitet werden.

10. Ultraschallwandler-Baugruppe nach Anspruch 4, wobei die Vias (76) durch die Isolierschicht (72) geleitet werden.

11. Ultraschallwandler-Baugruppe nach Anspruch 1, wobei die Wandlerelemente (54) kapazitive mikrobearbeitete Ultraschallwandlerelemente sind.

12. Ultraschallwandler-Baugruppe nach Anspruch 1, wobei das integrierte Schaltelement (60) eine anwendungsspezifische integrierte Schaltung ist.

13. Ultraschallwandler-Baugruppe nach Anspruch 1, bei der die Wandlerelemente (54) monolithisch auf dem integrierten Schaltelement (60) ausgebildet sind.

14. Ultraschallwandler-Baugruppe nach Anspruch 1, wobei das integrierte Schaltelement (60) und die Wandlerelemente (54) monolithisch auf einem Untergrund integriert sind.

15. Ultraschall-Bildgebungssystem (10) mit einer Ultraschallwandler-Baugruppe (50) nach Anspruch 1 zum Aussenden und Empfangen von Ultraschallwellen (56).

## Revendications

1. Ensemble transducteur à ultrasons (50), en particulier pour des systèmes d'imagerie à ultrasons (10), comprenant:
- un réseau de transducteurs (52) incluant une pluralité d'éléments transducteurs (54) pour émettre et recevoir des ondes ultrasonores (56), dans lequel chacun des éléments transducteurs (54) comprend une première électrode (66) connectée à une membrane flexible (64) et une seconde électrode (70),
- un dispositif à circuit intégré (60) connecté au réseau de transducteurs (52) pour commander les éléments transducteurs (54),
- dans lequel les premières électrodes (66) sont chacune connectées au moyen d'un vias (76) au dispositif de circuit intégré (60) et les vias (76) sont alimentés à travers les secondes électrodes (70), **caractérisé par le fait que** les secondes électrodes (70) sont connectées électriquement les unes aux autres et couplées à une alimentation en tension (78) pour fournir une tension de polarisation entre la première et la deuxième électrodes des éléments transducteurs (54), et les premières électrodes (66) étant couplées au dispositif à circuit intégré (60) pour fournir une tension d'attaque alternative à chacun des éléments transducteurs (54).

2. Ensemble transducteur à ultrasons selon la revendication 1, dans lequel les premières électrodes (66) sont des électrodes supérieures du réseau de transducteurs (52).

3. Ensemble transducteur à ultrasons selon la revendication 2, dans lequel les premières électrodes (66) sont chacune couplées à une surface supérieure de la membrane flexible respective (64).

4. Ensemble transducteur à ultrasons selon la revendication 1, dans lequel les secondes électrodes (70) sont des électrodes inférieures fixées à une couche d'isolation (72).

5. Ensemble transducteur à ultrasons selon la revendication 4, dans lequel la couche d'isolation (72) est fixée à une surface du dispositif à circuit intégré (60).

6. Ensemble transducteur à ultrasons selon la revendication 1, dans lequel les secondes électrodes (70) sont des électrodes rigides.

7. Ensemble transducteur à ultrasons selon la revendication 1, dans lequel une cavité (68) est respectivement formée entre chacune des premières électrodes et chacune des secondes électrodes.

8. Ensemble transducteur à ultrasons selon la revendication 1, dans lequel les membranes flexibles (64) des éléments transducteurs (54) sont chacune supportées au moyen d'une entretoise (74).

9. Ensemble transducteur à ultrasons selon la revendication 8, dans lequel les vias (76) pour connecter les premières électrodes (66) au circuit intégré (60) sont alimentés à travers les entretoises (74) .

10. Ensemble transducteur à ultrasons selon la revendication 4, dans lequel les traversées (76) sont alimentées à travers la couche d'isolation (72).

11. Ensemble transducteur à ultrasons selon la revendication 1, dans lequel les éléments transducteurs (54) sont des éléments transducteurs à ultrasons micro-usinés capacitifs.

12. Ensemble transducteur à ultrasons selon la revendication 1, dans lequel le dispositif de circuit intégré (60) est un circuit intégré spécifique à une application.

13. Ensemble transducteur à ultrasons selon la revendication 1, dans lequel les éléments transducteurs (54) sont formés de manière monolithique sur le dispositif à circuit intégré (60).

14. Ensemble transducteur à ultrasons selon la revendication 1, dans lequel le dispositif à circuit intégré (60) et les éléments transducteurs (54) sont intégrés de manière monolithique sur un substrat.

15. Système d'imagerie à ultrasons (10) comprenant un ensemble transducteur à ultrasons (50) selon la revendication 1 pour émettre et recevoir des ondes ultrasonores (56).
